(19) Europäisches Patentamt
European Patent Office
Office européen des brevets

(11) Veröffentlichungsnummer: **0 147 383**
**B1**

(12) # EUROPÄISCHE PATENTSCHRIFT

(45) Veröffentlichungstag der Patentschrift: **25.04.90**

(51) Int. Cl.$^5$: **A 61 K 6/04**

(21) Anmeldenummer: **84890237.5**

(22) Anmeldetag: **05.12.84**

(54) **Legierungspulvergemisch zur Dentalamalgam-Herstellung.**

(30) Priorität: **06.12.83 AT 4260/83**

(43) Veröffentlichungstag der Anmeldung:
**03.07.85 Patentblatt 85/27**

(45) Bekanntmachung des Hinweises auf die Patenterteilung:
**25.04.90 Patentblatt 90/17**

(84) Benannte Vertragsstaaten:
**BE CH DE FR GB IT LI LU NL SE**

(56) Entgegenhaltungen:
**US-A-2 281 991**
**US-A-3 841 860**
**US-A-3 997 328**

(73) Patentinhaber: **ÖGUSSA Österreichische Gold- und Silber-Scheideanstalt Scheid und Roessler Gesellschaft m.b.H. & Co. KG.**
**Gumpendorferstrasse 85**
**A-1060 Wien (AT)**

(72) Erfinder: **Stampach, Alfred, Dipl.-Ing.**
**Nussberggasse 15**
**A-1190 Wien (AT)**
Erfinder: **Gratzer, Gerhard**
**Schüttauplatz 19/14**
**A-1220 Wien (AT)**

(74) Vertreter: **Pfeifer, Otto, Dipl.-Ing. et al**
**Patentanwälte Dipl.-Ing. Dr. techn. Schütz, Alfred Dr. phil. Mrazek, Engelbert Dipl.-Ing. Holzer, Walter Dipl.-Ing. Pfeifer, Otto**
**Fleischmanngasse 9**
**A-1040 Wien (AT)**

**EP 0 147 383 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft Legierungspulvergemische aus zwei Komponenten zur Dentalamalgam-Herstellung, bei welchen die erste, durch mechanische Zerkleinerung hergestellte Komponente 65% oder mehr Silber, 29% oder weniger Zinn, 15% oder weniger Kupfer, 0 bis 3% Quecksilber und 0 bis 2% Zink enthält und die zweite, durch Verdüsen aus der Schmelze hergestellte Komponente unabhängig von deren chemischen Zusammensetzung stets Quecksilber enthält.

Es sind Einkomponenten-Legierungspulver zur Dentalamalgam-Herstellung bekannt, deren chemische Zusammensetzung der DIN 13904 (1980) bzw. der ÖNORM K 2702 (1982) mit mindestens 65% Silber, max. 29% Zinn, max. 15% Kupfer und max. 2% Zink entspricht. Derartige Einkomponenten-Legierungspulver haben sich seit langem in der Zahnheilkunde bewährt.

Legierungspulver mit dieser Zusammensetzung weisen nach dem Anmischen mit Quecksilber und Erhärten folgende vier Phasen auf:

$$Ag_3Sn\ (\gamma_0),\ Ag_3Hg_4\ (\gamma_1),\ Cu_3Sn\ (\varepsilon)$$

und

$$Sn_{7-8}Hg\ (\gamma_2).$$

Die $\gamma_2$-Phase ist die korrosionsanfälligste und mechanisch am wenigsten belastbare der vier Phasen. Das gesamte elektrochemische Potential einer Amalgamfüllung wird bei $\gamma_2$-Anwesenheit nachteilige beeinflußt. Lokale Expansion, Kantenbrüche und Sekundärkaries können die Folge sein (Mahler, Terkla, Eysden, Reisbick: Marginal fracture vs mechanical properties of amalgam, J. Dent. Res. 49 (1970); Till, Wagner, Untersuchungen zur Löslichkeit der Bestandteile von Amalgamfüllungen während des Kau- und Trinkaktes, Zahnärztl. Welt/Reform 82 (1973); K. Dreyer, Jϕrgensen, The mechanism of marginal fracture of amalgam fillings, Acta odont. scand. 23 (1965)).

Es ist bekannt, daß durch Mischungen von 50 bis 90% einer Pulversorte mit Kupfergehalten über 15% mit 10 bis 50% herkömmlicher Legierungen die nachteilige $\gamma_2$-Phase unterdrückt werden kann (US—A—3 841 860). Eine andere Möglichkeit, $\gamma_2$-freie Amalgame herzustellen, wird in der US—A—3 305 356 vorgeschlagen, wonach ein Zusatz von bis zu etwa 50% zinnfreiem Silber-Kupfer-Legierungspulver zu einem Silber-Zinn-Legierungspulver die $\gamma_2$-Abwesenheit im Amalgam bewirken soll.

Weitere Zweikomponenten-Legierungspulver zur Dentalamalgam-Herstellung sind aus der DE—A—30 18 376, DE—A—26 15 346 und der GB—A—1 513 988 bekannt.

Bei der Vorgangsweise nach den beiden US-Patentschriften wird erreicht, daß sich bei diesen Zweikomponentenmischungen beim Abbinden mit Quecksilber anstelle der nachteiligen $\gamma_2$-Phase die korrosionschemisch edlere und mechanisch stabilere $Cu_6Sn_5$ ($\eta'$)-Phase ring- bzw. schalenförmig um die kupferreiche Komponente ausbildet.

Es hat sich bei Leigerungspulvergemischen gemäß US—A—3 841 860 bzw. US—A—3 305 356 als nachteilig herausgestellt, daß die kupferreiche Komponente, die sich dem Quecksilber gegenüber an sich reaktionsträge verhält, durch Umwelteinflüsse und/oder unzureichende Aktivierung der Oberflächen, die bei 250 bis 350°C in reduzierender Atmosphäre erfolgt, zu Spaltbildungen zwischen kupferreicher und silberreicher Legierungskomponente Anlaß geben kann.

In Fig. 1 der angeschlossenen Zeichnungist diese legierungsinterne Spaltbildung bei den bekannten, $\gamma_2$-freien Amalgamen deutlich zu erkennen (Elektronenstrahl-Mikroanalyse ESMA, BSE-Bild, Vergrößerung 600:1).

Weiterhin haben vergleichende Ruhepotentialmessungen ergeben, daß diese auf eine unzureichende Reaktion der kupferreichen Komponente mit dem Quecksilber beim Anmischen und Erhärten des Amalgams zurückzuführenden Spaltbildungen eine nachhaltige Verschlechterung des elektrochemischen Potentials der gesamten Amalgamfüllung bewirken. In den Fig. 2 und 3 ist der Potentialverlauf sowohl für ein konventionelles $\gamma_2$-freies Zweikomponenten-Amalgam (Kurve B) als auch für ein erfindungsgemäßes Amalgam (Kurve A) aufgetragen. In Fig. 2 ist die logarithmische Ruhepotentialmessung ($U_o$) während 24 h dargestellt, ausgedrückt in Millivolt mV (gegen Normalwasserstoffelektrode), aufgetragen über logT; Alter der Proben: 3 Stunden vor Meßbeginn; Elektrolyt 0,2 M NaCl, pH 6,3. Figur 3 zeigt die Erfassung der Ruhepotentiale ($U_o$) während 6 Tagen (Alter der Proben: 27 Stunden vor Meßbeginn; Elektrolyt: 0,2 M NaCl, pH 6,3; Prüfkörper: 4 mm ϕ nach DIN 13 904).

Der Erfindung liegt daher die Aufgabe zugrunde, ein Legierungspulvergemisch der oben genannten Art derart weiterzuentwickeln, daß die korrosionsanfällige $\gamma_2$-Phase vermieden wird, ohne daß Spaltbildungen zwischen der kupferreichen Komponente und der silberreichen Matrix auftreten, die trotz Abwesenheit der $\gamma_2$-Phase die Korrosionsbeständigkeit einer Amalgam-Füllung wesentlich beeinträchtigen.

Eine weitere Zielsetzung der Erfindung ist es, ein Legierungspulvergemisch aus zwei Pulverkomponenten herzustellen, deren kupferreiche Komponente gegenüber atmosphärischen Einflüssen so unempfindlich sein soll, daß auch bei langfristiger Lagerung keine Spaltbildung zwischen kupferreicher Komponente und silberreicher Matrix durch Passivierung der Oberfläche der kupferreichen Komponente auftreten kann.

Diese Aufgabe wird erfindungsgemäß durch ein Legierungspulvergemisch zur Herstellung von Dentalamalgam, bestehend aus einer durch mechanische Zerkleinerung hergestellten ersten Silber-Zinn-Kupfer-Komponente und einer zweiten, durch Verdüsen aus der Schmelze hergestellten Silber-Kupfer-Komponente mit höherem Kupferanteil als die erste Komponente gelöst, welches Legierungspulvergemisch sich dadurch

auszeichnet, daß sich die erste Komponente aus 65 bis 70% Silber, 29 bis 26% Zinn, 15 bis 2% Kupfer, 0 bis 3% Quecksilber und 0 bis 2% Zink zusammensetzt, eine Teilchengröße bis zu 80 µm aufweist und in einem Anteil von 40 bis 80% vorliegt, während die zweite Komponente sich aus 40 bis 80% Silber, 20 bis 60% Kupfer, 1 bis 9% Quecksilber und gegebenenfalls 1 bis 8% Zinn und/oder 0 bis 2% Zink zusammensetzt und gleichfalls eine Teilchengröße bis zu 80 µm aufweist. Die kupferreiche zweite, durch Verdüsen aus der Schmelze erhaltene Komponente enthält somit, unabhängig von deren Zusammensetzung, stets Quecksilber.

Es hat sich gezeigt, daß durch das erfindungsgemäße Einbringen von Quecksilber in die kupferreiche, durch Verdüsen aus der Schmelze hergestellte zweite Komponente die Reaktion der kupferreichen Komponente mit dem Quecksilber beim Anmischen und Erhärten des Amalgams vollständig verläuft. Spaltbildungen zwischen kupferreicher Komponente und silberreicher Matrix sind auch bei langfristiger Lagerung unter ungünstigen Bedingungen nicht nachweisbar, vgl. Fig. 4, die die Spaltfreiheit eines erfindungsgemäß aufgebauten Amalgams zeigt (ESMA, BSE-Bild, Vergrößerung 600:1). Amalgame, die aus erfindungsgemäßen Legierungspulvern hergestellt werden, sind frei von $\gamma_2$-Phase und verhalten sich, wie die Messungen des Ruhepotentials ergaben, elektrochemisch wesentlich positiver, da im Gegensatz zu den herkömmlichen Legierungspulvergemischen keinerlei Spaltbildungen zwischen kupferreicher Komponente und silberreicher Matrix auftreten. Die bei konventionellen Amalgamen gegebene Korrosionsgefahr kann somit mit den erfindungsgemäß ausgebildeten Amalgamen vermieden werden.

Ein weiterer Vorteil der Erfindung liegt darin, daß sich—wie durch Untersuchungen mittels Elektronenstrahl-Mikroanalyse nachgewiesen werden konnte—durch das in der kupferreichen Komponente bereits enthaltene Quecksilber und der damit verbundenen Reaktionsfähigkeit auch bei hohen Kupfergehalten der durch Verdüsen aus der Schmelze gewonnenen Komponente die für die Unterdrückung der $\gamma_2$-Phase notwendige $\eta'$-Phase rascher ausbildet.

Beispiel 1:

67% einer Komponente 1 mit der chemischen Zusammensetzung 70% Silber, 27% Zinn und 3% Kupfer werden mechanisch zerkleinert und auf eine Teilchengröße ≤60 µm ausgesiebt und mit 33% einer durch Verdüsen aus der Schmelze hergestellten Komponente 2 mit der chemischen Zusammensetzung 68% Silber, 27% Kupfer und 5% Quecksilber und einer Teilchengröße 40≤ µm vermischt. Die elektrochemischen Eigenschaften eines aus diesem Legierungspulvergemisch hergestellten Amalgams sind in Fig. 2 und 3, Kurven A, veranschlaulicht.

Beispiel 2:

70% einer Komponente 1 mit der chemischen Zusammensetzung 69,5% Silber, 26% Zinn, 3% Kupfer und 1,5% Quecksilber werden mechanisch zerkleinert und auf eine Teilchengröße ≤80 µm ausgesiebt und mit 30% einer durch Verdüsen aus der Schmelze hergestellten Komponente 2 mit der chemischen Zusammensetzung 60% Silber, 33% Kupfer, 1% Zinn und 6% Quecksilber und einer Teilchengröße ≤33 µm vermischt.

Beispiel 3:

50% einer Komponente 1 mit der chemischen Zusammensetzung 70% Silber, 27% Zinn, 2% Kupfer und 1% Quecksilber werden mechanisch zerkleinert und auf eine Teilchengröße von ≤80 µm ausgesiebt und mit 50% einer durch Verdüsen aus der Schmelze hergestellte Komponente 2 mit der chemischen Zusammensetzung 70% Silber, 26% Kupfer und 4% Quecksilber mit einer Teilchengröße ≤60 µm vermischt.

Die als Beispiele angeführten Mischungen ergeben Amalgame guter Konsistenz und Stopfbarkeit, sie sind frei von $\gamma_2$-Phase und weisen auch nach langfristiger Lagerung des Legierungspulvers keinerlei die Korrosionsbeständigkeit beeinträchtigenden Spaltbildungen auf.

In ihrer chemischen Zusammensetzung entsprechen die als Beispiel angeführten Mischungen ebenso wie in ihren physikalischen Eigenschaften der DIN 13 904 (1980) bzw. der ÖNORM K 2702 (1982).

**Patentanspruch**

Legierungspulvergemisch zur Herstellung von Dentalamalgam, bestehend aus einer durch mechanische Zerkleinerung hergestellten ersten Silber-Zinn-Kupfer-Komponente und einer zweiten, durch Verdüsen aus der Schmelze hergestellten Silber-Kupfer-Komponente mit höherem Kupferanteil als die erste Komponente, dadurch gekennzeichnet, daß sich die erste Komponente aus

—65 bis 70% Silber
—29 bis 26% Zinn,
—15 bis 2% Kupfer,
— 0 bis 3% Quecksilber und
— 0 bis 2% Zink

zusammensetzt, eine Teilchengröße bis zu 80 µm aufweist, und in einem Anteil von 40 bis 80% vorliegt, während die zweite Komponente sich aus

—40 bis 80% Silber,
—20 bis 60% Kupfer,
— 1 bis 9% Quecksilber und gegebenenfalls
— 1 bis 8% Zinn und/oder
— 0 bis 2% Zink

zusammensetzt und gleichfalls eine Teilchengröße bis zu 80 µm aufweist.

**Revendication**

Mélange de poudres d'alliages pour la préparation d'un amalgame dentaire, consistant en un premier composant argent-étain-cuivre préparé par broyage mécanique et en un deuxième composant argent-cuivre préparé par atomisation de la masse fondue et ayant une teneur en cuivre plus élevée que le premier composant, caractérisé en ce que le premier composant est constitué par

—65 à 70% d'argent
—29 à 26% d'étain
—15 à 2% de cuivre
— 0 à 3% de mercure, et
— 0 à 2% de zinc

a une granulomètrie jusqu'à 80 µm et est présent en une proportion de 40 à 80%, tandis que le deuxième composant est constitué par

—40 à 80% d'argent
—20 à 60% de cuivre
— 1 à 9% de mercure et éventuellement
— 1 à 8% d'étain et/ou
— 0 à 2% de zinc

et a également une granulométrie jusqu'à 80 µm.

**Claim**

Alloy powder mixture for preparing dental amalgam, consisting of a first silver-tin-copper component prepared by mechanical comminution and a second silver-copper component showing a higher copper content than the first component, prepared by melt spraying, characterized in that the first component is comprised of

—65 to 70% silver,
—29 to 26% tin,
—15 to 2% copper
— 0 to 3% mercury and
— 0 to 2% zinc,

has a particle size up to 80 µm and is present in an amount of 40 to 80%, whereas the second comonent is comprised of

—40 to 80% silver,
—20 to 60% copper,
— 1 to 9% mercury and optionally
— 1 to 8% tin and/or
— 0 to 2% zinc

and also has a particle size up to 80 µm.

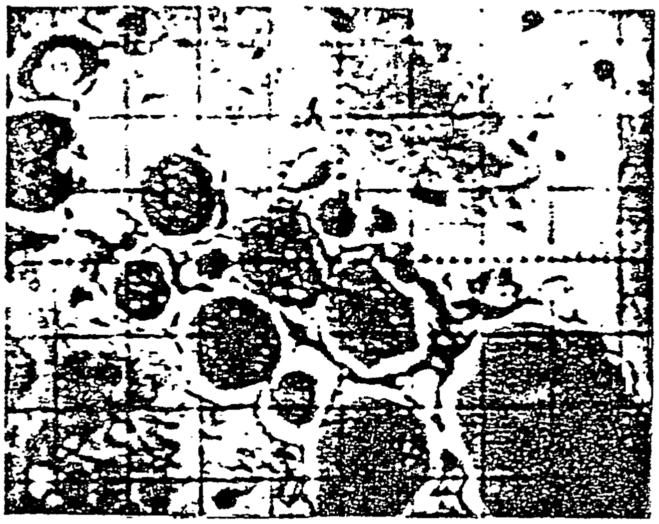

Fig. 1 V = 600:1

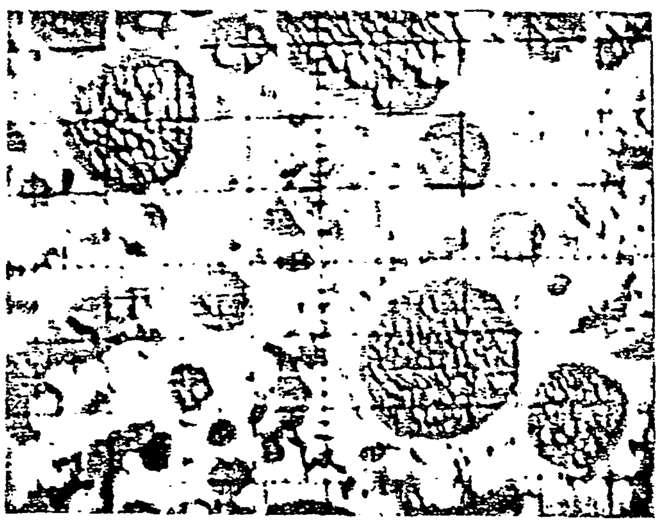

Fig. 4 V = 600:1

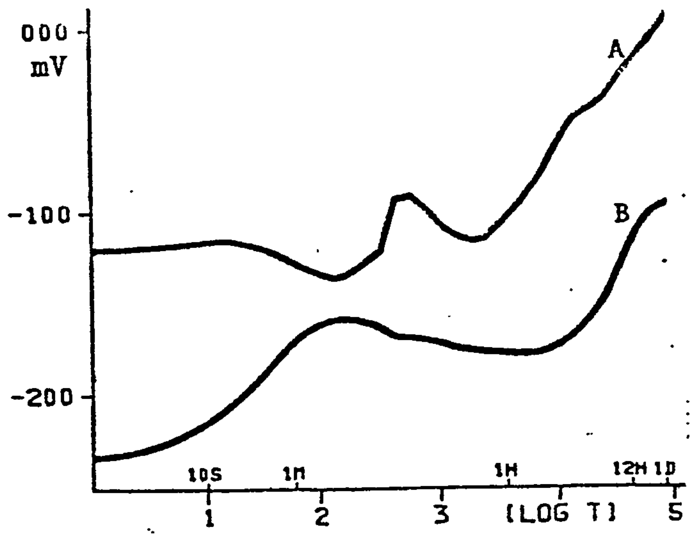


Fig. 2

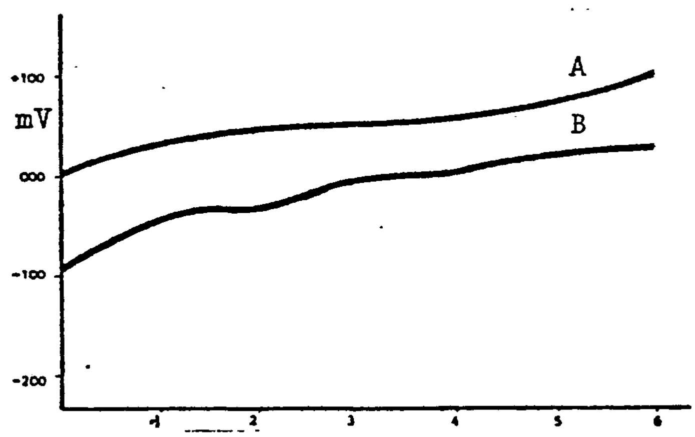


Fig. 3

Zeit (in Tagen)

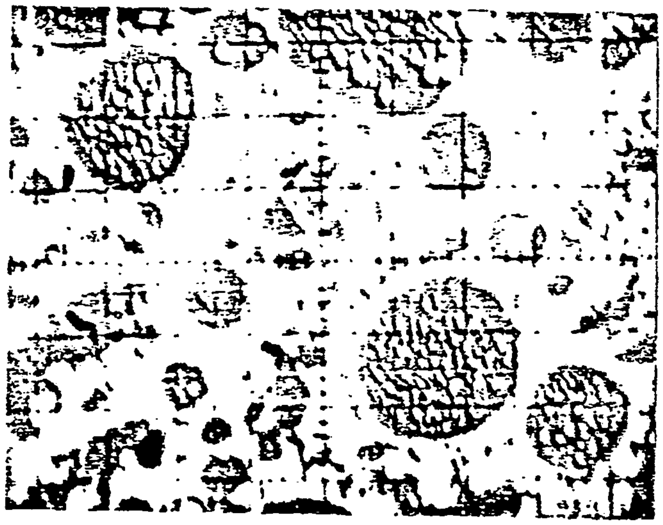

Fig. 4 V = 600:1